# EUROPEAN PATENT APPLICATION

(11) **EP 3 485 891 A1**
(43) Date of publication of application: **22.05.2019**
(21) Application number: 17827846.1
(22) Date of filing: 30.06.2017
(51) Int. Cl.: A61K 31/7048, A61K 36/185, A61K 9/12, A61K 9/06

(54) **WOUND TREATMENT COMPOSITION CONTAINING ULDAVIOSIDE A COMPOUND**

(30) Priority: 12.07.2016 KR 20160087822; 13.06.2017 KR 20170074274
(71) Applicant: Elcubio Co., Ltd., Yuseong-gu, Daejeon 34054 (KR)
(72) Inventor: SEO, Won Ho, Daejeon 34344 (KR); KIM, Ik Hwi, Suwon-si Gyeonggi-do 16689 (KR); CHA, Byung Yoon, Busan 49228 (KR); KIM, Ho, Uiwang-si Gyeonggi-do 16049 (KR)
(74) Representative: Adam, Holger
(86) International application number: PCT/KR2017/006931
(87) International publication number: WO 2018/012772

(57) **Abstract**

The present invention relates to a wound treatment composition containing as an active ingredient an uldavioside A compound separated from an Ulmus davidiana Planch. extract. The uldavioside A compound shows excellent efficacy for wound treatment and thus can be utilized as a therapeutic agent for treating wounds.

## Description

### Technical Field

The present invention relates to a wound treatment composition containing, as an active ingredient, an uldavioside A compound isolated from an extract of the root bark of an elm tree *(Ulmus davidiana* Planch).

### Background Art

The wound indicates damage to skin tissue, which protects the body from external invasion and maintains moisture and body temperature, and wound healing is an essential response for the functional and morphological restoration of damaged tissues. If a wound is not properly dressed, the interior organs of the body may be fatally damaged due to bacterial infection or the like, and therefore, in the event of a wound, it is important to take measures so that the damaged skin region can be restored to have a similar structure to the original skin as quickly as possible.

Wound healing is composed of three stages: hemostasis and inflammation phases as a first stage, a proliferation phase as a second stage, and a remodeling phase as a third stage, the respective stages being continuously carried out in an overlapping manner (Bello Y.M., et al., 2000; Midwood K.S., et al., 2004; and Stadelmann W.K., et al., 1998).

In the event of a wound, hemostasis and inflammation occur as the first stage. In the hemostasis phase, platelets gather at a wound site to form fibrin clots within several minutes, serving to prevent bleeding (Midwood K.S., et al., 2004; and Sandeman S.R., et al., 2000). In the inflammation response, bacteria and tissue debris are removed by phagocytosis, and several cytokines are released from macrophages, aiding in the migration and mitosis of differentiating cells at the proliferation stage.

In the proliferation phase, which is the second stage, angiogenesis, collagen accumulation, granulation tissue formation, epithelialization, wound contraction, and the like occur (Midwood K.S., et al., 2004; and Chang H.Y., et al., 2004). This phase is an initial step for wound adhesion and collagenous fiber rearrangement, and fibroblasts appear at the wound site in this phase (Byl N.N., et al., 1992).

In the remodeling phase, spaces in the wound are completely replaced with granulation tissues, angiogenesis reaches its peak, and collagenous fibers are abundant, thus gradually restoring the normal thickness of epithelial cells, and finally completing wound healing (Galeano M., et al., 2008; Garg H.G., et al., 2000).

If disrupted tissues, foreign substances, debris of necrotic tissues, and the like are not removed by inflammatory cells at the inflammation phase during the stages of wound healing, the wound healing is delayed at the proliferation phase in the next stage, and therefore the role of the inflammation phase is very important (Enoch P., et al., 2004).

Specifically, in the response occurring in the inflammation phase, local blood vessels contract in the event of tissue damage, and then the blood vessels expand as histamine, bradykinin, prostaglandin, serotonin, norepinephrine, and the like are released in the wound site. The expanded blood vessels form gaps between endothelial cells, and plasma and leukocytes escape into the interstitial fluid between such gaps. Within several hours after the damage to tissues, polymorphonuclear leukocytes are influenced by complements to migrate toward the wound site, and phagocytize the damages tissue debris and bacteria at the site. Macrophages also arrive at the damage site within several hours from wound occurrence, and perform a phagocytosis action. The activated macrophages produce several cytokines and reactive oxygen species (ROS) to thus activate nuclear factor-κB (NF-κB), which is a transcriptional factor in the inflammation response. As a result, the expressions and activities of inducible nitric oxide synthase (iNOS) and cyclooxygenase-2 (COX-2) are increased and nitric oxide (NO) and prostaglandin E2 (PGE₂) are excessively produced, resulting in the inflammation response. In addition, the reactive oxygen species produced from the macrophages removes bacteria to thus prevent infection at the wound site, and act as an intercellular second messenger, playing an essential role in wound healing. However, it has been known that excessive production of the reactive oxygen species causes oxidative stress around the wound site to thus regulate the expressions of inflammatory mediators, thereby influencing the inflammation response in wound healing, thus delaying a wound healing rate. Therefore, an attempt has been made to find an antioxidative substance inhibiting the excessive production of reactive oxygen species (Eunkyo Park, 2011).

*The root bark of an elm tree* is a dried product of stem and root barks obtained by peeling cork layers from an elm tree *(Ulmus davidiana* Planch, *Ulmus davidiana* var. *japonica* Nakai) belonging to the family *Ulmaceae.* It has been reported in oriental medicine that the root bark of an elm tree is sweet and nontoxic, is used for swellings, arthritis, gastric ulcer, gastroenteritis, and the like, and also has superior effects on phlegm, cancer, wounds, and inflammation (Duke J.A., 1985). The natural substances present in the root bark of an elm tree are β-sitosterol, phytosterol, stigmasterol, tannin, starch, polysaccharides, and the like. Many studies have been conducted about the isolation (Matsuzaki T., et al., 1985) and chemical composition (Duke J.A., 1985) of friedelin, epifriendelalol, and taraxerol, which are painkilling ingredients, but studies on the diverse physiological activities of the root bark of an elm tree have not been attempted.

While studying a wound treatment composition containing an extract of the root bark of an elm tree, the present inventors verified that an uldavioside A compound isolated from an extract of the root bark of an elm tree had excellent wound healing efficacy, and therefore completed the present invention.

Korean Patent Publication No. 2000-0041190 as a prior art discloses the prevention and treatment of a pharmaceutical composition, containing the cortex of an elm tree, on diseases, such as inflammation and wounds; and Korean Patent Publication No. 2004-0050154 as a prior art discloses a wound treatment composition containing an extract of the root of an elm tree, but such patents are different from the present invention since the uldavioside A compound is not disclosed. Korean Patent Registration No. 0893166 discloses the fibroblast proliferating effect and the antioxidative effect of an extract of an elm tree, but such a patent is different from the present invention since the uldavioside A compound and wound healing are not disclosed.

Furthermore, a non-patent document by Son B.W., et al. discloses an uldavioside A compound isolated from an elm tree, but does not disclose a wound healing effect.

### Detailed Description of the Invention

### Technical Problem

An aspect of the present invention is to provide a wound treatment composition containing, as an active ingredient, an uldavioside A compound isolated from an extract of the root bark of an elm tree *(Ulmus davidiana* Planch).

### Technical Solution

In accordance with an aspect of the present invention, there is provided a wound treatment composition containing an uldavioside A compound of chemical formula 1 below as an active ingredient:

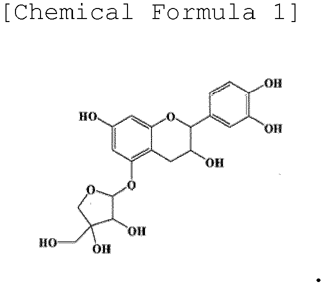

The wound may be selected from the group consisting of hacks, bedsores, burns, abrasions, puncture wounds, ulcerative wounds, stabs, chronic skin wounds caused by reactive oxygen species, bruises, cuts, throat or mouth mucosal wounds, lacerations, diabetic ulcers, lower limb ulcers, hypertension ischemic ulcers, venous ulcers, and foot ulcers.

The uldavioside A compound may be contained in a content of 0.001-50 wt% relative to the total weight of the composition.

The composition may be a pharmaceutical composition formulated as an oral administration, a spray, a gel, an ointment, a cream, or an externally-applied preparation.

Hereinafter, the present invention will be described in detail.

The present inveniton is directed to a wound treatment composition containing an uldavioside A compound of chemical formula 1 below as an active ingredient:

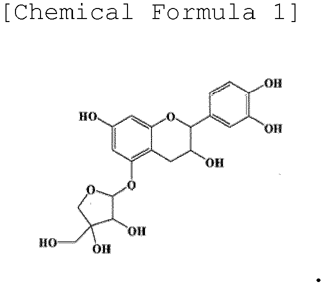

The uldavioside A compound of chemical formula 1 can be synthesized by a conventional method, can be prepared as a pharmaceutically acceptable salt, or can be isolated and purified from an extract of the root bark of elm tree.

The root bark of an elm tree is a dried product of stem and root barks obtained by peeling cork layers from an elm tree *(Ulmus davidiana* var. *japonica* Nakai, *Ulmus davidiana* Planch) belonging to the family Ulmaceae.

The extract of the root bark of an elm tree can be extracted from the root bark of an elm tree by using, as a solvent, water, a C1-C4 lower alcohol, or a mixture solution thereof. The C1-C4 lower alcohol may be selected from the group consisting of methanol, propanol, isopropanol, butanol, and iso-butanol. Preferably, the extraction is carried out using water.

The compound isolated from the extract of the root bark of an elm tree can be purified using column chromatography. The chromatography can be selected from silica gel column chromatography, HP-20 column chromatography, RP-18 column chromatography, LH-20 column chromatography, high-performance liquid chromatography, and the like.

The wound treatment composition is to heal a damaged wound of skin tissues, and the wound is selected from the group consisting of hacks, bedsores, burns, abrasions, puncture wounds, ulcerative wounds, stabs, chronic skin wounds caused by reactive oxygen species, bruises, cuts, throat or mouth mucosal wounds, lacerations, diabetic ulcers, lower limb ulcers, hypertension ischemic ulcers, venous ulcers, and foot ulcers.

The diabetic ulcer may be a diabetic foot ulcer.

The composition is provided as a pharmaceutical composition containing the uldavioside A compound of chemical formula 1 and an excipient. The uldavioside A compound may be contained in 0.001-50 wt%, preferably 0.001-40 wt%, and more preferably 0.001-30 wt% relative to the total weight of the composition.

The pharmaceutical composition may be formulated as an oral dosage form, such as a powder, granules, a tablet, a capsule, a suspension, an emulsion, a syrup, an aerosol, or a spray, an external preparation, a suppository, and a sterile injectable solution, according to a conventional method for each form. A carrier, an excipient, and a diluent that may be contained in the composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and a mineral oil. The composition may be formulated using a diluent or an excipient, such as a filler, an extender, a binder, a wetting agent, a disintegrant, or a surfactant. A solid preparation for oral administration includes a tablet, a pill, a powder, granules, a capsule, and the like. These types of solid preparations may be prepared by mixing the uldavioside A compound of the present invention with at least one excipient, for example, starch, calcium carbonate, sucrose or lactose, gelatin, or the like. Alternatively, lubricants such as magnesium stearate and talc may be used in addition to simple excipients. A liquid preparation for oral administration corresponds to a suspension, a liquid for internal use, an emulsion, a syrup, and the like, and may include simple diluents that are frequently used, such as water and liquid paraffin, and several types of excipients, such as a wetting agent, a sweetener, a fragrance, and a preservative. A preparation for parenteral administration includes a sterilized aqueous solution, a non-aqueous solvent, a suspension, an emulsion, a freeze-drying agent, and a suppository. The non-aqueous solvent and the suspension may include propylene glycol, polyethylene glycol, vegetable oil such as olive oil, an injectable ester such as ethyl oleate, and the like. A substrate for the suppository may include Witepsol, Macrogol, tween-61, cacao butter, laurin butter, or glycerogelatin.

The wound treatment composition may be a pharmaceutical composition formulated as an oral administration, a spray, a gel, an ointment, a cream, or an externally-applied preparation, but is not limited thereto.

The dose of the pharmaceutical composition of the present invention may vary depending on the age, gender, and body weight of the subject to be treated, the particular disease or pathological condition to be treated, the severity of the disease or pathological condition, the route of administration, and the determination of a prescriber. The determination of the dose based on these factors is within the level of a person skilled in the art, and a usual dose is in a range of 0.01-2000 mg/kg/day. A more preferable dose is 1-500 mg/kg/day. The dosing may be carried out once a day or several times a day. The above dose is not intended to restrict the scope of the present invention in any way.

The pharmaceutical composition of the present invention may be administered to mammals, such as rats, mice, cattle, and humans, through various routes. The manner of administration can be anticipated, for example, the administration may conducted orally, rectally, or through intravenous, intramuscular, subcutaneous, endometrial, or intracerebroventricular injection, or via application to the skin. The compound of the present invention has little toxicity and side effects, and thus the compound is a medicine that can be safely used even for long-term administration for preventive purposes.

### Advantageous Effects

The present invention is directed to a wound treatment composition containing, as an active ingredient, an uldavioside A compound isolated from an extract of the root bark of an elm tree, and it has been verified that the uldavioside A compound has wound healing efficacy.

It is expected through the results that the uldavioside A compound of the present invention can be easily used as a wound healing medicine for, for example, hacks, bedsores, burns, abrasions, puncture wounds, ulcerative wounds, stabs, chronic skin wounds caused by reactive oxygen species, bruises, cuts, throat or mouth mucosal wounds, lacerations, diabetic ulcers, lower limb ulcers, hypertension ischemic ulcers, venous ulcers, and foot ulcers.

### Brief Description of the Drawings

FIG. 1 shows ¹H-¹H COSY (bold lines) and ¹H-¹³C HMBC correlations (arrows) of the uldavioside A compound of the present invention.
FIG. 2 shows cytotoxic results of the uldavioside A compound of the present invention on C2C12 cells (A) and NIH3T3 cells (B).
FIG. 3 shows wound healing effects of the uldavioside A compound of the present invention on inflammation stress in C2C12 cells (A and B) and NIH3T3 cells (C and D).
FIG. 4 shows wound healing effects of the uldavioside A compound of the present invention on glucose oxidative stress in C2C12 cells (A and B) and NIH3T3 cells (C and D).
FIG. 5 shows a hack wound healing effect of the uldavioside A compound of the present invention in animal models. (A) shows an image of a wound site and (B) shows a quantification graph of the wound site, according to the treatment with the uldavioside A compound.
FIG. 6 shows observation results of histological changes according to the hack healing by the uldavioside A compound of the present invention in animal models. (A) shows H & E stain results and (B) shows Masson's trichrome stain results.
FIG. 7 shows the results confirming the expression levels of (A) VEGF and CD31, which are proteins associated with angiogenesis, and (B) bFGF and β-catenin, which are proteins associated with tissue regeneration, according to wound healing by the uldavioside A compound of the present invention in animal models.
FIG. 8 shows cytoprotective effects of the uldavioside A compound of the present invention against glucose oxidative stress in a high-glucose environment on HDF cell (A) and C2C12 cell (B).
FIG. 9 shows (A) an image of a wound site and (B) quantification results of the wound site size according to the circular cut healing of the uldavioside A compound of the present invention in diabetes-induced animal models.

### Mode for Carrying Out the Invention

Hereinafter, preferable embodiments of the present invention will be described in detail. However, the present invention is not limited to the embodiments described herein, and thus may be embodied in many different forms. Rather, these embodiments are provided so that this disclosure will be thorough and complete, and will fully convey the scope of the invention to those skilled in the art.

### <Example 1: Isolation of uldavioside A compound>

The root bark of an *elm tree (Ulmus davidiana* Planch), which had been dried by a farm, was purchased and used. An extract of the root bark of an elm tree was extracted by adding 1 L of purified water to 10 g of the dried root bark of an elm tree and repeating, three times, treatment using a semi-continuous type low-temperature vacuum extractor at 25°C for 12 hours, and was then subjected to impurity removal using a filter paper to give a water extract of the root bark of an elm tree. The water extract of the root bark of an elm tree was subjected to Diaion HP-20 column chromatography in isocratic elution conditions with increasing polarity in the order of 30% methanol, 50% methanol, 70% methanol, and 100% methanol, thereby giving three fractions (Fr. E-1 to E-3). Among the fractions, the Fr. E-1 was subjected to Sephadex LH-20 column chromatography according to the isocratic dissolution conditions using 70% methanol as an isocratic solvent, thereby isolating an uldavioside A compound (9 mg).

### <Example 2: Investigation of physical and chemical structures of uldavioside A compound>

Uldavioside A;
Light brown powder;
¹H NMR and ¹³C NMR data being shown in Table 1 below;
ESI-MS [M+H]⁺ *m*/*z* 423.1, C₂₀H₂₂O₁₀.

**[Table 1]**

| Position | Uldavioside A compound | |
|---|---|---|
| | δ_{C}(ppm)^{a} | δ_{H} (ppm)^{b} |
| 2 | 82.9 | 4.59 (1H, d. *J* = 6.8 Hz) |
| 3 | 68.6 | 3.98 (1H, m) |
| 4 | 28.4 | 2.84 (1H, dd, *J* = 16.5. 5.5 Hz) |
| | | 2.53 (1H, dd, *J* = 16.5, 8.3 Hz) |
| 4a | 103.2 | |
| 5 | 158.2 | |
| 6 | 97.2 | 6.12 (1H, d, *J* = 2.0 Hz) |
| 7 | 157.6 | |
| 8 | 96.8 | 6.06 (1H, d, *J* = 2.0 Hz) |
| 8a | 156.9 | |
| 9 | 132.1 | |
| 10 | 115.2 | 6.82 (1H, d, *J* = 2.0 Hz) |
| 11 | 146.2 | |
| 12 | 146.3 | |
| 13 | 116.1 | 6.75 (1H, dd, *J* = 8.2 Hz) |
| 14 | 119.9 | 6.70 (1H, dd. *J* = 8.2, 2.0 Hz) |
| 1' | 108.7 | 5.47 (1H, d, *J* = 3.4 Hz) |
| 2' | 78.2 | 4.13 (1H, d, *J* = 2.7 Hz) |
| 3' | 80.3 | |
| 4' | 75.4 | 4.07 (1H, d. *J* = 9.6 Hz) |
| | | 3.84 (1H, d, *J* = 9.6 Hz) |
| 5' | 64.9 | 3.60 (2H. m) |

| | | |
|---|---|---|
| ^{a 13}C NMR (150 MHz in CD₃OD) spectroscopy data ^{b 1}H NMR (600 MHz in CD₃OD) spectroscopy data | | |

### <Experimental Example 1: Incubation of animal cells>

In order to investigate the activity of the uldavioside A compound of the present invention, animal cells were incubated. Here, C212 cells, as myoblasts, and NIH3T3 cells and human diploid fibroblasts (HDFs), as fibroblasts, were used as the animal cells. Each type of cells were incubated in a 5% CO₂ incubator containing Dulbecco's modified eagle's medium (DMEM) supplemented with 10% fetal bovine serum (FBS), 100 U/mℓ penicillin, and 100 *µ*g/mℓ streptomycin at 37 °C.

### <Experimental Example 2: Investigation of cytotoxicity of uldavioside A compound>

The cytotoxicity of the uldavioside A compound of the present invention was investigated using a 3-(4,5-dimethylthiazol-2-yl)-2,5-diphenyltetrazolium bromide (MTT) assay.

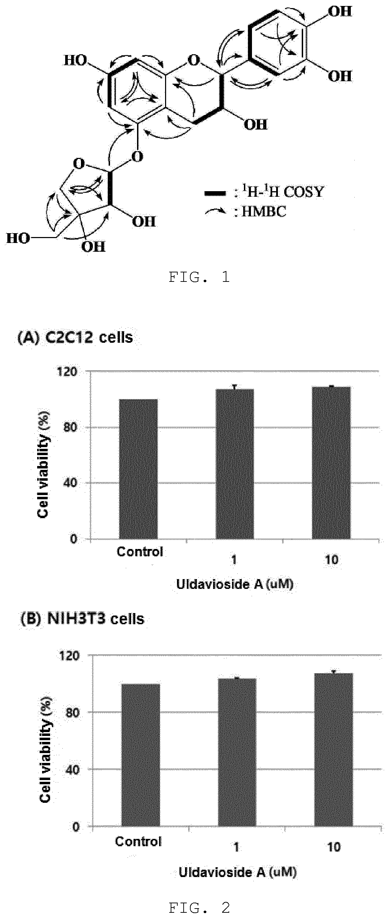

The C2C12 and NIH3T3 cells incubated in Experimental Example 1 above were dispensed at 1×10⁵ cells per well in a 48-well plate and then incubated for 24 hours. Thereafter, DMEM medium containing 0.5% FBS, 100 U/mℓ penicillin, and 100 *µ*g/mℓ streptomycin was placed therein, and then the cells were incubated for 18 hours. After the 18-hour incubation, the cells were treated with an uldavioside A compound at 1 µM and 10 µM and then incubated for 24 hours. Here, non-treated C2C12 and NIH3T3 cells were used as controls. After 24 hours, the cells were treated with an MTT solution at 100 *µ*g/mℓ and incubated at 37 °C for 3 hours. Thereafter, the culture liquid was removed and the formed formazan precipitate was dissolved in 200 *µ*ℓ of dimethyl sulfoxide (DMSO), and then the absorbance was measured at 570 nm. The results are shown in FIG. 2.

As shown in FIG. 2, the cell viability increased in the C2C12 cells (2A) and NIH3T3 cells (2B) treated with the uldavioside A compound, compared with the controls.

It could be seen through the results that the uldavioside A compound of the present invention showed no cytotoxicity.

### <Experimental Example 3: Investigation of wound healing effect of uldavioside A compound>

In order to investigate a wound healing effect according to the treatment with the uldavioside A compound of the present invention, a wound healing assay was conducted.

### Experimental Example 3-1: Investigation of healing effect on wound caused by inflammatory stress

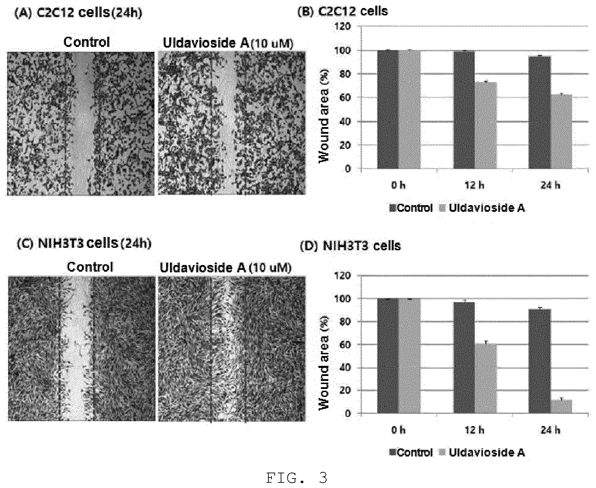

The C2C12 and NIH3T3 cells incubated in Experimental Example 1 above were dispensed at 1×10⁶ cells per well in a 12-well plate, and then stabilized through incubation for 24 hours. Thereafter, DMEM medium containing 0.5% FBS, 100 U/mℓ penicillin, and 100 *µ*g/mℓ streptomycin was placed therein, and then the cells were incubated for 18 hours. After the 18-hour incubation, a scratch with about 0.9 mm was made in the center of each well with growing cells, and the cells were treated with a 10 µM uldavioside A compound, followed by pre-incubation for 1 hour. After 1 hour, the cells were treated with 10 *µ*g/mℓ lipopolysaccharide (LPS) for inflammatory stress stimulation, and then migration of the cells was observed while the cells were incubated. Here, at 0, 12, and 24 hours, the cells were fixed with a fixative (2% formaldehyde and 0.2% glutaraldehyde) and stained with 0.3% crystal violet, and then, the migration of the cells was checked using a microscope, and the reduction in area due to the migration of the cells into a wounded site. The results are shown in FIG. 3. Here, the controls were stimulated with inflammatory stress using LPS, without pre-incubation with the uldavioside A compound.

As shown in FIG. 3, the cell migration of C2C12 cells (3A and 3B) and NIH3T3 cells (3C and 3D) for inflammatory stress increased when the cells were treated with the uldavioside A compound of the present invention compared with the controls, and especially, the migration activity of the NIH3T3 cells was higher.

### Experimental Example 3-2: Investigation of healing effect on wound caused by glucose oxidative stress

Diabetes is a disease in which complications occur most frequently due to oxidative stress during the degradation of glucose in the blood. Glucose oxidase produces hydrogen peroxide (H₂O₂) during the degradation of glucose in cells, causing glucose oxidative stress. Therefore, the healing effect of the uldavioside A compound of the present invention on a wound due to glucose oxidative stress was verified.

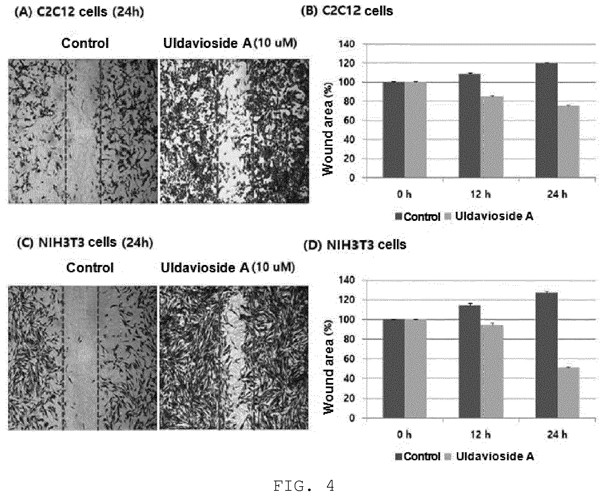

An experiment was conducted by the same method as in Experimental Example 3-1 above, and 7 mU/mℓ glucose oxidase, instead of LPS, was used for treatment in order to cause glucose oxidative stress. The results are shown in FIG. 4.

As shown in FIG. 4, the cell migration of C2C12 cells (4A and 4B) and NIH3T3 cells (3C and 3D) for glucose oxidative stress increased when the cells were treated with the uldavioside A compound of the present invention, compared with the controls, and especially, the migration activity of the NIH3T3 cells was higher.

It could be seen through the results in FIGS. 3 and 4 that the uldavioside A compound of the present invention had excellent healing effects on wounds due to inflammatory stress and glucose oxidative stress.

### <Experimental Example 4: Investigation of wound healing effect of uldavioside A compound>

### Experimental Example 4-1: Investigation of wound healing effect through visual observation

In order to investigate a wound healing effect of the uldavioside A compound of the present invention, an animal experiment was conducted.

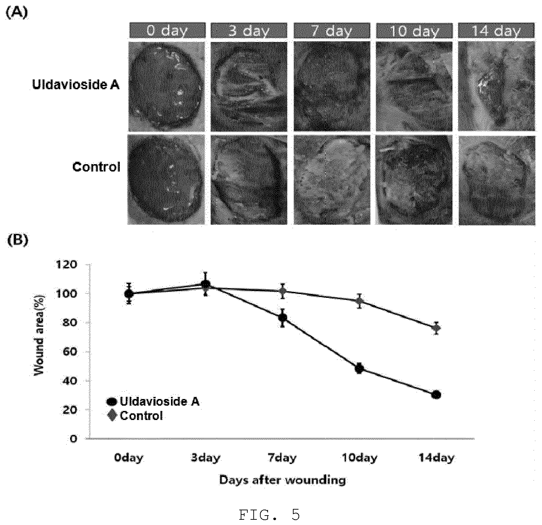

A hind leg of 7-8 week-old Sprague-Dawley white rats was shaved, and disinfected with 70% alcohol, and then a 3 cm-diameter circular full-thickness hack was made. After the full-thickness hack was made, the rats were randomly assigned and divided into a control treated without the uldavioside A compound of the present invention and a treatment group. For the treatment group, on the day of hack induction, the wound was treated with an ointment (Preparation Example 1-2 below) containing 0.025 wt% of the uldavioside A compound, wrapped with sterilized Korean traditional paper, and fixed with a band and a bandage to maintain a wet environment. For the control, the wound was treated with an ointment not containing the uldavioside A compound. Thereafter, on days 3, 7, 10, and 14 of wound induction, the wound site was observed, and the size of the wound site was quantified. The results are shown in FIG. 5.

As shown in the image of the wound site (5A) and the quantification graph of the wound site (5B) in FIG. 5, the wound site started healing from day 7, and almost healed on day 14 in the group treated with the uldavioside A compound, compared with the control.

It could be seen through the results that the uldavioside A compound of the present invention has an excellent hack wound healing effect.

### Experimental Example 4-2: Observation of histological change by wound healing

In order to investigate a hack healing effect of the uldavioside A compound of the present invention, a histological change was observed using the hack-induced animal tissue.

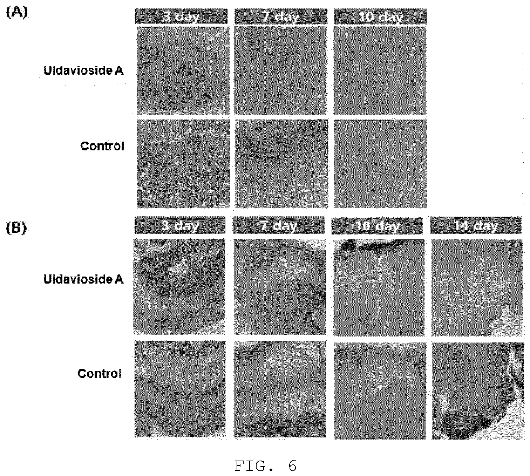

After the hack healing effect was observed in Experimental Example 4-1 above, the rats were sacrificed, and the muscle tissue of the wound site was collected through biopsy. The collected tissue was fixed by treatment with 10% formalin for 24 hours. The fixed tissue was embedded with paraffin, and cut into a 5 *µ*m thickness to obtain tissue sections. The tissue sections were deparaffinized, hydrated, and washed, and then subjected to hematoxylin & eosin stain or Masson's trichrome stain. The histological change associated with wound healing was analyzed using a microscope, and the results are shown in FIG. 6.

As shown in the H&E stain results in FIG. 6(A), the inflow of immune cells was reduced on day 3 after hack induction, angiogenesis and granulation tissues formation were actively progressing on day 7, and the active proliferation of fibroblasts were observed on day 10 in the group treated with the uldavioside A compound. In the control, compared with the group treated with the uldavioside A compound, the inflow of immune cells was higher on day 3 after hack induction, which continued until day 7. On day 10, the granulation tissue appeared, and the proliferation of fibroblasts was not observed.

Masson's trichrome stain is used to investigate collagen in the tissue, and collagenosis means that the wound in the dermis healed and the functions of the tissue were restored.

As shown in the Masson's trichrome stain results in FIG. 6(B), the group treated with the uldavioside A compound showed a remarkably increased amount of collagen stained on day 7 after hack induction compared with the control, and showed collagenosis in most tissues on day 17.

It could be seen through the results that the uldavioside A compound of the present invention has an excellent wound healing effect.

### Experimental Example 4-3: Investigation of changes of proteins associated with angiogenesis and tissue regeneration by hack healing

In order to investigate a hack healing effect of the uldavioside A compound of the present invention, the expression changes of proteins associated with angiogenesis and tissue regeneration were observed.

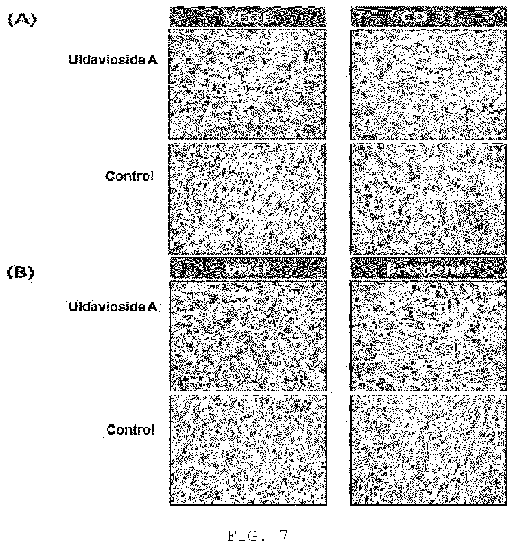

The tissue sections on day 10, which were fabricated in Experimental Example 4-2 above, were deparaffinized, hydrated, and washed, and were then subjected to immunohistochemistry (IHC) using antibodies of vascular endothelial growth factor (VEGF) and CD31, which are proteins associated with angiogenesis in tissue, and basic fibroblast growth factor (bFGF) and β-catenin, which are proteins associated with tissue regeneration. The expressions of the respective proteins were analyzed, and the results are shown in FIG. 7.

As shown in the immunohistochemistry results in FIG. 7, it was verified that the expression levels of the angiogenesis-associated proteins VEGF and CD31 (7A) and the tissue regeneration-associated proteins bFGF and β-catenin (7B) significantly increased in the group treated with the uldavioside A compound, compared with the control.

It could be seen through the results that the uldavioside A compound of the present invention promoted angiogenesis and tissue regeneration occurring during wound healing, thereby showing an excellent wound healing effect.

### <Experimental Example 5: Investigation of diabetic ulcer healing effect of uldavioside A compound>

### Experimental Example 5-1: Investigation of cytoprotective activity of uldavioside A compound against glucose oxidative stress

The cytoprotective activity of the uldavioside A compound of the present invention against glucose oxidative stress was investigated.

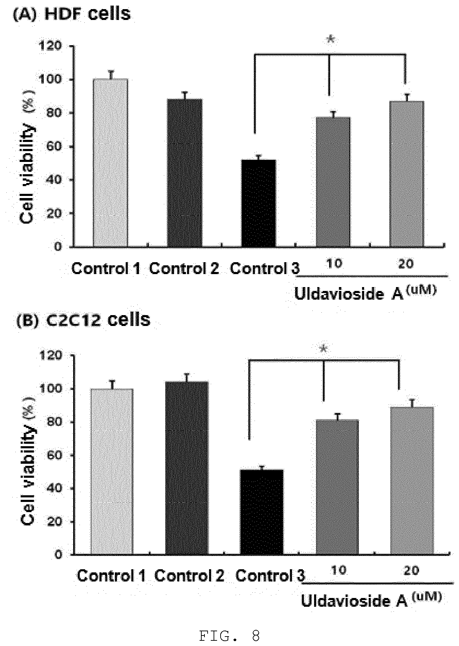

The C2C12 cells and HDF cells incubated in Experimental Example 1 were dispensed at 1×10⁵ per well in a 48-well plate, respectively, and then incubated for 24 hours. Thereafter, each well was treated with 50 mM D-glucose to create a high-glucose environment. Then, the cells were treated with the uldavioside A compound at 10 µM and 20 µM, and after 1 hour, were treated with 7 mU/mℓ glucose oxidase, followed by incubation for 24 hours. Here, non-treated C2C12 or HDF cells were used as control 1, high-glucose state cells were used as control 2, and high-glucose state cells treated with glucose oxidase were used as control 3. After 24 hours, the cells were treated with an MTT solution at 100 *µ*g/mℓ and incubated at 37 °C for 3 hours. Thereafter, the culture liquid was removed and the formed formazan precipitate was dissolved in 200 *µ*ℓ of DMSO, and then the absorbance was measured at 570 nm. The results are shown in FIG. 8.

As shown in FIG. 8, it was verified that both of the HDF cells (8A) and C2C12 cells (8B) showed reduced cell viability due to glucose oxidative stress by glucose oxidase in the high-glucose environment (control 3), while the reduction in cell viability by glucose oxidative stress was inhibited in a dose-dependent manner when the cells were treated with the uldavioside A compound of the present invention.

It could be seen through the results that the uldavioside A compound has a cytoprotective effect against glucose oxidative stress in a high-glucose environment, and thus it can be predicted that the uldavioside A compound of the present invention can effectively heal wounds in diabetic patients having a slow healing rate.

### Experimental Example 5-2: Investigation of diabetic ulcer healing effect of uldavioside A compound

In order to investigate a diabetic ulcer healing effect of the uldavioside A compound of the present invention, an animal experiment was conducted.

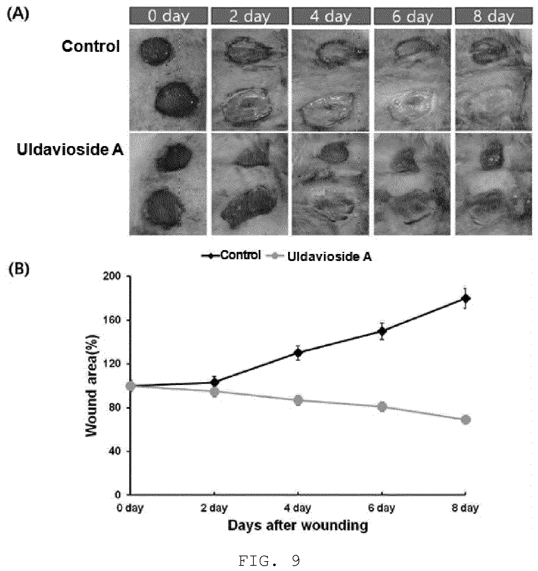

7-week-old Sprague-Dawley white rats with a body weight of about 300 g were intraperitoneally injected with 60 mg/kg streptozotocin to induce diabetes. After three days, only white rates with a blood glucose level of 300 mg/dℓ or higher were selected by collecting venous blood from the tail vein and measuring the glucose level using a blood glucose meter. The dorsal part of each of the selected white rats was shaved, and a circular cut with a diameter of 1 or 1.5 cm was made on the dorsal part 4-5 cm away from the ear using a puncher. After the circular cut was made, the white rats were randomly assigned and divided into a control treated without the uldavioside A compound of the present invention and a treatment group. For the treatment group, on the day of circular cut induction, the cut was treated with an ointment containing 0.025 wt% of the uldavioside A compound (Preparation Example 1-2 below), wrapped with sterilized Korean traditional paper, and fixed with a band and a bandage to maintain a wet environment. For the control, the cut was treated with an ointment not containing the uldavioside A compound. Thereafter, the wound site was observed for 8 days after circular cut induction at intervals of 2 days, and the size of the wound site was quantified. The results are shown in FIG. 9.

As shown in the image of the wound site (9A) and the quantification graph of the wound site size (9B) of the circular cut in the diabetes-induced animal models in FIG. 9, it was verified that the wound site started healing from day 4 and the wound size remarkably decreased in the group treated with the uldavioside A compound, compared with the control.

It could be seen through the results that the uldavioside A compound of the present invention has an excellent diabetic ulcer healing effect.

### <Preparation Example 1: Pharmaceutical preparation>

### Preparation Example 1-1: Gel dosage form pharmaceutical preparation

In a preparation container, 15 wt% of Pluronic F-127 (a BASF Co. product) was placed in pH 6.0 buffer, and the mixture was slowly stirred, and then allowed to stand at 4°C for 20 hours, thereby obtaining a viscous liquid. In a separate container, 0.0025 wt% of the uldavioside A compound of the present invention was placed in a pH 6.0 buffer, and dissolved with stirring, and then the mixture was introduced into the preparation container, followed by stirring. In another separate container, 4 wt% of ethanol, 0.1 wt% of p-hydroxybenzoic acid methyl ester, and 0.1 wt% of p-hydroxybenzoic acid propyl ester were introduced, and dissolved with stirring, and then the resultant mixture was introduced into the preparation container. The introduced mixture was stirred for 20 minutes, and allowed to stand for 4-5 hours in a refrigerator, thereby obtaining a bright yellow viscous liquid. The obtained viscous liquid was slowly heated until a gel was obtained therefrom.

### Preparation Example 1-2: Ointment-formulation pharmaceutical preparation

In a preparation container, 15 wt% of white petrolatum, 10 wt% of cetostearyl alcohol, 7 wt% of hard liquid paraffin, and 1.5 wt% of Cetomacrogol were melted with heating to about 65 °C, and then the molten liquid was filtered through a 100-mesh sieve. In a separate container, purified water was poured, and 0.025 wt% of the uldavioside A compound of the present invention was placed, and dissolved with stirring, and then the resultant mixture was introduced into the preparation container. In another separate container, 0.12 wt% of p-hydroxybenzoic acid methyl ester and 0.08 wt% of p-hydroxybenzoic acid propyl ester were dissolved in boiling purified water at 95 °C, and then the resultant mixture was introduced into the preparation container. The introduced mixture was stirred for 20 minutes, and emulsified using a homogenizer at 2500 rpm for 20 minutes. Upon completion of the emulsification, the resultant emulsion was cooled with slow stirring.

### Preparation Example 1-3: Preparation of tablet

200 g of the uldavioside A compound of the present invention was mixed with 175.9 g of lactose, 180 g of potato starch, and 32 g of colloidal silicic acid. A 10% gelatin solution was added to the mixture, pulverized, and passed through a 14-mesh sieve. The resultant product was dried, and 160 g of potato starch, 50 g of an active ingredient, and 5 g of magnesium stearate were added thereto to obtain a mixture, which was then prepared into tablets.

## Claims

1. A wound treatment composition containing an uldavioside A compound of chemical formula 1 below as an active ingredient:

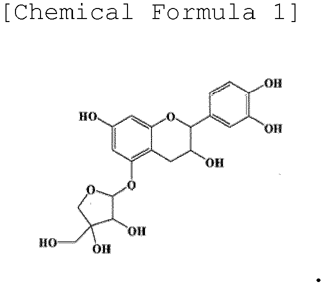

2. The wound treatment composition of claim 1, wherein the wound is selected from the group consisting of hacks, bedsores, burns, abrasions, puncture wounds, ulcerative wounds, stabs, chronic skin wounds caused by reactive oxygen species, bruises, cuts, throat or mouth mucosal wounds, lacerations, diabetic ulcers, lower limb ulcers, hypertension ischemic ulcers, venous ulcers, and foot ulcers.

3. The wound treatment composition of claim 1, wherein the uldavioside A compound is contained in a content of 0.001-50 wt% relative to the total weight of the composition.

4. The wound treatment composition of any one of claims 1 to 3, wherein the composition is a pharmaceutical composition formulated as an oral administration, a spray, a gel, an ointment, a cream, or an externally-applied preparation.
